# EUROPÄISCHE PATENTSCHRIFT

(11) **EP 0 729 946 B1**
(45) Veröffentlichungstag und Bekanntmachung des Hinweises auf die Patenterteilung: **15.12.1999**
(21) Anmeldenummer: 96810103.0
(22) Anmeldetag: 22.02.1996
(51) Int. Cl.: C07D 211/46, C07D 211/58, C08K 5/3435, C09K 15/30, C08G 63/685

(54) **Kondensate aus butinverknüpften gehinderten Aminen**
Condensates of butyne-linked hindered amines
Condensates des amines encombrées liées par butyne

(30) Priorität: 02.03.1995 CH 59695
(43) Veröffentlichungstag der Anmeldung: 04.09.1996
(73) Patentinhaber: Ciba Specialty Chemicals Holding Inc., 4057 Basel (CH)
(72) Erfinder: Steinmann, Alfred, Dr., 1724 Praroman (CH)

(56) Entgegenhaltungen:
- EP-A- 0 388 535
- EP-A- 0 685 465
- US-A- 4 386 127

## Beschreibung

Gegenstand der Erfindung sind neue oligomere oder polymere Verbindungen, welche durch Kondensation von 1,4-Bis-(4-hydroxy-2,2,6,6-tetramethylpiperidin-1-yl)-but-2-in oder entsprechenden Diaminen mit geeigneten Reaktionspartnern erhalten werden können, deren Verwendung zum Stabilisieren von organischem Material gegen oxidativen, thermischen oder aktinischen Abbau, sowie entsprechende stabilisierte Zusammensetzungen und ein Verfahren zum Stabilisieren.

Eine Reihe von wissenschaftlichen Veröffentlichungen beschreiben 1-Butinyl-2,2,6,6-tetramethylpiperidine, deren Piperidinring in 4-Stellung unsubstituiert ist, und deren mögliche Anwendung als Arzneimittel; so M. E. Zuhair et al., Eur. J. Med. Chem. 27, 93 (1992) (C.A.117:26405v); J.M.A. Al-Rawi et al. in Org. Magn. Reson. 19, 91 (1982) (C.A.97:155132w) und Org. Magn. Reson. 15, 285 (1981) (C.A.95:60789k); B. Karlen et al., J. Med. Chem. 13, 651 (1970) (C.A.73:44857w).

1,6-Bis(2,2,6,6-tetramethylpiperidin-1-yl)-hexadiin, dessen Piperidinringe in 4-Stellung unsubstituiert sind, wird in den Publikationen US-A-3755586 (C.A.80:19550c); W. B. Lutz et al., J. Org. Chem. 27, 1695 (1962); und US-A-3085093 (C.A.59:9998e) als pharmazeutisch wirksame Verbindung beschrieben.

Es wurde nun gefunden, daß sich gewisse Derivate von butinverknüpften gehinderten Aminen zu kettenförmigen Molekülen umsetzen lassen, die sich überraschenderweise sehr gut zum Einsatz als Stabilisatoren für organisches Material eignen.

Gegenstand der Erfindung sind daher zunächst neue Verbindungen der Formel I 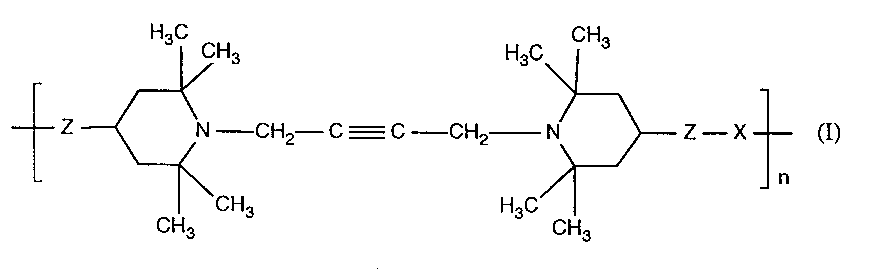
worin n eine Zahl aus dem Bereich 1 bis 100 ist;
X die Bedeutungen -R⁷-, -CO-, -CO-CO-, -CO-R²-CO-, -CO-NH-R⁵-NR-CO- und -CO-O-R⁶-O-CO- und 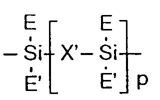 umfaßt, wobei p eine Zahl aus dem Bereich 0-6, X' O oder eine direkte Bindung, E C₁-C₁₂-Alkyl, Phenyl, C₇-C₁₂-Phenylalkyl oder C₅-C₁₂-Cycloalkyl bedeutet, und E' Wasserstoff ist oder eine der für E gegebenen Bedeutungen hat;
Z im Fall, daß X R⁷ ist, O oder NR¹ ist; Z im Fall, daß 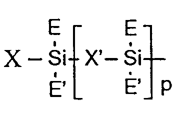 ist, O bedeutet; und
Z in allen anderen Fällen für O, NH oder NR¹ steht;
R¹ C₁-C₈-Alkyl, C₇-C₉-Phenylalkyl, C₅-C₇-Cycloalkyl, C₂-C₈-Alkanoyl, Benzoyl oder durch C₁-C₄-Alkyl substituiertes Benzoyl darstellt;
R² und R⁷ C₁-C₁₈-Alkylen; durch O oder S unterbrochenes C₂-C₁₈-Alkylen; Phenylen; einen gemischt aromatisch/aliphatischen Rest enthaltend 7 bis 12 Kohlenstoffatome; Naphthylen; oder einen Rest -R⁴-D-R⁴- bedeuten;
R⁴ 1,4-Cyclohexylen oder 1,4-Phenylen; und
R⁶ Phenylen, Naphthylen oder 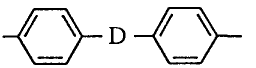 bedeutet;
R⁵ im Fall, daß Z O ist, eine der für R² angegebenen Bedeutungen hat; und
R⁵ im Fall, daß Z NH oder NR¹ ist, eine der für R⁶ angegebenen Bedeutungen hat; und
D C₁-C₃-Alkylen, -S-,-SO-, -SO₂-,-CO-, -O-, -NH- oder -NR¹- darstellt.

Die Absättigung der Kettenenden in Formel I ergibt sich aus den Herstellungsbedingungen, die weiter unten beschrieben werden. In der Regel weisen die Verbindungen der Formel I am Z-terminalen Ende die Endgruppe A und am X-terminalen Ende die Endgruppe B auf und entsprechen damit der Formel Ia 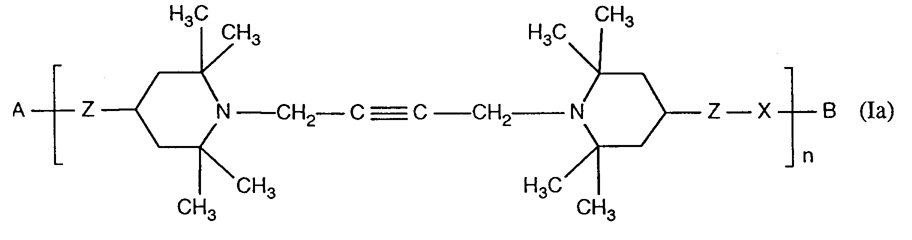
wobei n, X und Z die oben angegebenen Bedeutungen haben;
A Wasserstoff oder eine Endgruppe der Formel -X-Z' ist;
B eine Endgruppe der Formel 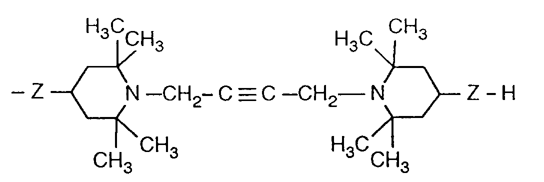
oder -Z' ist; und
Z' OH, NH₂ oder OR¹ bedeutet.

Mit Alkylen wird hier allgemein ein polyvalenter, insbesondere ein bivalenter, gesättigter aliphatischer Kohlenwasserstoffrest bezeichnet, wobei die beiden Bindungen an demselben oder an verschiedenen Kohlenstoffatomen lokalisiert sein können.

R¹ bedeutet als C₁-C₈-Alkyl Methyl, Ethyl, Propyl, Butyl, Pentyl, Hexyl, Heptyl oder Octyl; als C₇-C₉-Phenylalkyl z.B. Benzyl, 1-Phenylethyl, 2-Phenylethyl; oder als C₅-C₇-Cycloalkyl Cyclopentyl, Cyclohexyl oder Cycloheptyl.

R¹ bedeutet als Rest einer organischen Carbonsäure beispielsweise Acetyl, Benzoyl, Propanoyl, Butanoyl, Pentanoyl, Hexanoyl, Heptanoyl, Octanoyl; bevorzugt ist Acetyl oder Benzoyl, vor allem Acetyl.

R² und R⁷ als C₁-C₁₈-Alkylen umfassen Methylen, Ethylen, Propylen, Butylen, Pentylen, Hexylen, Heptylen, Octylen, Nonylen, Decylen, Undecylen, Dodecylen, Tridecylen, Tetradecylen, Pentadecylen, Hexadecylen, Heptadecylen oder Octadecylen. Bevorzugt ist geradkettiges Alkylen. Ebenfalls bevorzugt ist C₁-C₁₂-Alkylen. Besonders bevorzugt ist geradkettiges C₂-C₁₂-Alkylen.

R² und R⁷ als durch O oder S unterbrochenes C₂-C₁₈-Alkylen bedeuten beispielsweise (CH₂)ₘ-S-(CH₂)ⱼ oder (CH₂)ₘ-O-(CH₂)ⱼ, wobei die Summe der ganzen Zahlen m + j eine Zahl aus dem Bereich 2-18 ist; Beispiele sind -CH₂-S-CH₂- oder -CH₂-O-CH₂-.

R² und R⁷ in der Bedeutung gemischt aromatisch/aliphatischer Rest enthaltend 7 bis 12 Kohlenstoffatome umfaßt zweibindige Kohlenwasserstoffreste, welche sowohl mindestens eine gesättigte aliphatische Einheit als auch eine Aryleinheit enthalten, wobei die Bindungen entweder beide an der Aryleinheit, oder beide an einer aliphatischen Einheit oder je eine Bindung an der Aryl- und eine an einer aliphatischen Einheit lokalisiert sind. Unter Aryleinheiten sind in wesentlichen Phenyl, Phenylen, Naphthyl oder Naphthylen zu verstehen. Die genannten Reste umfassen daher beispielsweise solche der Formeln 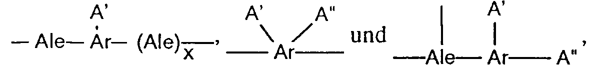 wobei Ar jeweils eine Aryleinheit ist wie angegeben, Ale C₁-C₆-Alkylen, A' und A'' unabhängig voneinander H oder C₁-C₆-Alkyl sind und der Index x 0 oder 1 ist, mit der Maßgabe, daß die Summe der Kohlenstoffatome 7 bis 12 beträgt.

Bevorzugte solche Reste sind u.a. 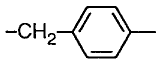 (1,4-Tolylen), 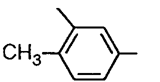 (2,4-Tolylen), 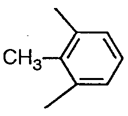 (2,6-Tolylen), 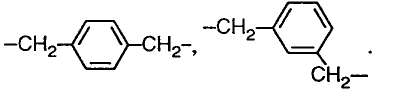
E ist besonders bevorzugt Phenyl oder Methyl, E' H, Phenyl oder Methyl.
Z ist bevorzugt O oder NH, besonders O.
X ist bevorzugt -CO-, -CO-CO-, -CO-R²-CO- oder -CO-NH-R⁵-NH-CO- oder 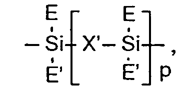 vor allem -CO-,-CO-CO- oder -CO-R²-CO-.
Vorzugsweise ist p 0 oder 1.
R² ist bevorzugt Phenylen, CH₂-S-CH₂, CH₂-O-CH₂, 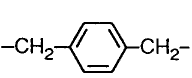 oder C₁-C₁₂-Alkylen, besonders geradkettiges C₁-C₁₂-Alkylen.

Im Fall Z = O ist R⁵ bevorzugt C₃-C₁₂-Alkylen, Phenylen, Naphthylen, Cyclohexylen, Tolylen, durch Phenylen unterbrochenes Ethylen, oder -R⁴-CH₂-R⁴-; besonders bevorzugt sind geradkettiges C₄-C₈-Alkylen, Phenylen, 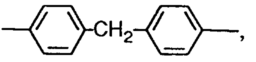 2,4-Tolylen, 2,6-Tolylen, 1,5-Naphthylen, 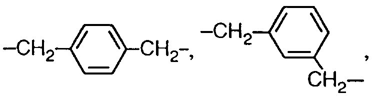 1,4-Cyclohexylen, 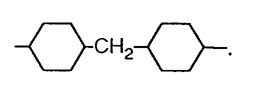
R⁵ und R⁶ stellen vorzugsweise Phenylen dar.
R⁷ ist vorzugsweise Alkylen, besonders C₁-C₁₂-Alkylen;
D ist bevorzugt -O-,-CO-, -NH- oder NR¹, besonders -CO-;
   von besonderem technischem Interesse ist auch die Bedeutung -CH₂-.

Die Verbindungen der Formel I können beispielsweise oligomere oder auch polymere Verbindungen sein. Häufig handelt es sich um Verbindungsgemische mit unterschiedlichem Polymerisationsgrad. Einen bevorzugten Gegenstand der Erfindung bilden Verbindungen der Formel I, worin n eine ganze Zahl aus dem Bereich 4 bis 80, besonders aus dem Bereich 5 bis 50, vor allem 10 bis 30 ist.

Ebenfalls bevorzugt sind Verbindungen der Formel I, worin
X die Bedeutungen -CO-,-CO-CO-, -CO-R²-CO-, 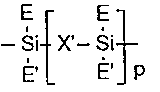 und -CO-NH-R⁵-NH-CO- umfaßt, wobei p 0 oder 1 ist, X' O oder eine direkte Bindung, E' Wasserstoff, Phenyl oder Methyl und E Phenyl oder Methyl darstellt;
R¹ C₁-C₈-Alkyl, Benzyl, C₂-C₈-Alkanoyl, Benzoyl oder Cyclohexyl darstellt;
R² und R⁷ C₁-C₁₈-Alkylen; durch O oder S unterbrochenes C₂-C₁₈-Arkylen; Phenylen; einen gemischt aromatisch/aliphatischen Rest enthaltend 7 bis 12 Kohlenstoffatome; Naphthylen; oder einen Rest -R⁴-D-R⁴-;
R⁴ 1,4-Cyclohexylen oder 1,4-Phenylen;
R⁵ im Fall, daß Z O ist, C₃-C₁₂-Alkylen, Phenylen, Naphthylen, Cyclohexylen, Tolylen, durch Phenylen unterbrochenes Ethylen, oder -R⁴-CH₂-R⁴- bedeutet; und
D C₁-C₃-Alkylen, -NH-, -NR¹-, -CO- oder -S- darstellt.

Besonders bevorzugt sind Verbindungen der Formel I, worin
Z für O oder NR¹ steht;
X die Bedeutungen -CO-CO-, -CO-R²-CO-, 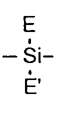 und -CO-NH-R⁵-NH-CO- umfaßt;
R² und R⁷ C₁-C₁₈-Alkylen; Phenylen, CH₂-S-CH₂, CH₂-O-CH₂ oder 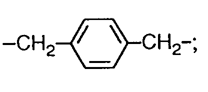 und
R⁵ im Fall, daß Z O ist, C₃-C₁₂-Alkylen, Phenylen, Naphthylen, Cyclohexylen, Tolylen, durch Phenylen unterbrochenes Ethylen, oder -R⁴-CH₂-R⁴- darstellt, wobei R⁴ die oben angegebenen Bedeutungen umfaßt; und R⁵ im Fall, daß Z NR¹ ist, Phenylen bedeutet; vor allem solche, worin n eine ganze Zahl aus dem Bereich 5 bis 50;
Z ein Sauerstoffatom bedeutet;
R² und R⁷ C₁-C₁₂-Alkylen oder Phenylen; und
R⁵ C₃-C₁₂-Alkylen, Phenylen, Naphthylen oder Cyclohexylen ist.

Verbindungen der Formel I gehören beispielsweise zu folgenden Klassen von Verbindungen:
1. Polyester, wenn Z für O steht und X die Bedeutung -CO-R²-CO- hat;
2. Polyurethane, wenn Z für O steht und X die Bedeutung -CO-NH-R⁵-NH-CO- hat, oder wenn Z für NH oder NR¹ steht und X die Bedeutung -CO-O-R⁶-O-CO- hat;
3. Polyamide, wenn Z für NH oder NR¹ steht und X die Bedeutung -CO-R²-CO- hat;
4. Polyether, wenn Z für O steht und X die Bedeutung -R⁷- hat;
5. Polycarbonate, wenn Z für O steht und X die Bedeutung -CO- oder -CO-O-R⁶-O-CO-hat;
6. Polyamine, wenn Z für NR¹ steht und X die Bedeutung -R⁷- hat;
7. Polyharnstoffe, wenn Z für NH oder NR¹ steht und X die Bedeutung -CO-NH-R⁵-NH-CO- oder -CO- hat;
8. Polysilane oder -siloxane, wenn X für die Formel 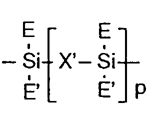 steht.

Bevorzugt sind Polyester, Polyether und Polyurethane.

Zur Herstellung von Verbindungen der Formel I, worin Z die Bedeutung O hat, geht man vielfach von einem Diol der Formel II aus:

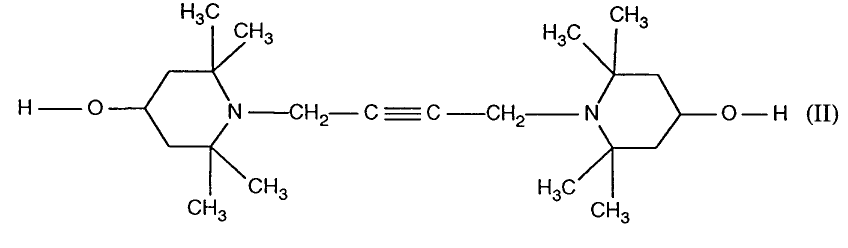

Die Verbindung der Formel II kann in Analogie zu bekannten Herstellungsverfahren erhalten werden, wie sie beispielsweise in den Publikationen US-A-3085093; J. Org. Chem. 27, 1695 (1962); Eur. J. Med. Chem. 27, 93 (1992); US-A-4386127; oder J. Med. Chem. 13, 651 (1970); und in der dort zitierten Literatur beschrieben sind. So kann 2,2,6,6-Tetramethyl-4-hydroxy-piperidin mit Butinylhalogeniden oder -dihalogeniden oder zunächst mit Propargylhalogenid umgesetzt werden. Als Halogenide werden häufig die Chloride und insbesondere die Bromide eingesetzt. Erhaltenes 1-Propargyl-2,2,6,6-tetramethyl-4-hydroxy-piperidin kann mit Formaldehyd und weiterem 2,2,6,6-Tetramethyl-4-hydroxy-piperidin zum disubstituierten Butin umgesetzt werden.

Bevorzugt wird die Verbindung der Formel II hergestellt, indem ein Ester einer aromatischen Sulfonsäure der Formel III 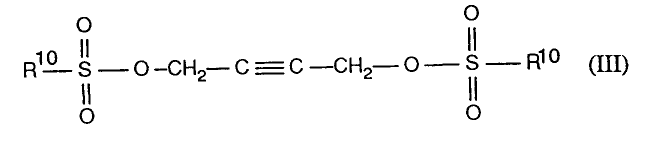 worin R¹⁰ Phenyl oder durch C₁-C₈-Alkyl, C₁-C₈-Alkoxy oder Halogen substituiertes Phenyl ist, mit einer Verbindung der Formel IV 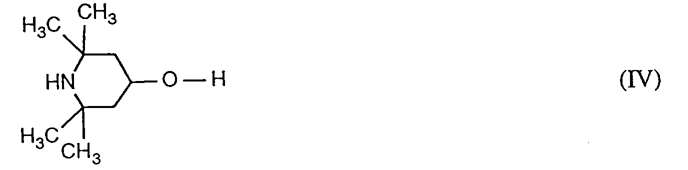 umgesetzt wird.
R¹⁰ ist vorzugsweise Phenyl, Tolyl, Chlorphenyl oder Methoxyphenyl; insbesondere Phenyl oder Tolyl. Besonders bevorzugt werden in diesem Verfahren als Edukt der Formel III 2-Butin-1,4-diol-ditosylat eingesetzt.

Zweckmäßig werden etwa 2 Äquivalente der Verbindung der Formel IV pro Äquivalent der Verbindung der Formel III eingesetzt. Vorteilhaft kann die Verbindung der Formel IV auch im Überschuß verwendet werden, beispielsweise in einer Menge von 1,5-4, besonders 2-3 Äquivalenten der Verbindung der Formel IV pro Sulfonyloxygruppe in Verbindung der Formel III; das nicht umgesetzte Edukt kann in diesen Fall vom Produkt abgetrennt und erneut in die Reaktion eingesetzt werden.

Die Umsetzung erfolgt zweckmäßig in einem inerten Lösungsmittel. Dies sind häufig polare organische Lösungsmittel wie z.B. halogenierte Kohlenwasserstoffe, Ester, Ether, Ketone, Amide, Nitrile, tert. Amine oder Sulfoxide; geeignet sind beispielsweise Dimethylformamid, Tetrahydrofuran, Dioxan, Chloroform, Triethylamin, Pyridin, Dibutylether, Dimethylsulfoxid, Acetonitril; besonders bevorzugt ist Acetonitril.

Die Temperatur kann während der Reaktion im Bereich von 0 bis 200°C liegen, zweckmäßig beträgt die Temperatur 20-160°C, vor allem 40-140°C.

Die Temperatur der Reaktionsmischung kann für die Dauer der Reaktion im Siedebereich gehalten werden (Rückfluß). Dazu wird eine Lösemittel enthaltende Reaktionsmischung, im Allgemeinen unter Normaldruck, zum Siedepunkt erwärmt und das verdampfte Lösemittel mit Hilfe eines geeigneten Kühlers kondensiert und wieder der Reaktionsmischung zugeführt.

Die Reaktion kann unter Ausschluß von Sauerstoff durchgeführt werden, beispielsweise durch Spülen mit einem Inertgas wie Argon; Sauerstoff stört jedoch nicht in jedem Fall, so daß die Reaktion auch ohne die genannte Maßnahme durchgeführt werden kann.

Nach beendeter Reaktion kann die Aufarbeitung nach gängigen Methoden erfolgen; zweckmäßig wird die Mischung zunächst mit Wasser verdünnt, beispielsweise indem das Reaktionsgemisch in das 1-4-fache Volumen Eiswasser gegeben wird; anschließend kann das Produkt direkt abgetrennt oder extrahiert werden, zur Extraktion eignet sich z.B. Essigester oder Chloroform. Wird extrahiert, so kann das Produkt in üblicher Weise durch Entfernen des Lösemittels isoliert werden; dies geschieht zweckmäßig nach Trocknen der organischen Phase. Möglich sind auch das Einschalten weiterer Reinigungsschritte wie beispielsweise Waschen mit wässriger NaCl-Lösung, Dispergieren von Aktivkohle, Filtrieren, Umkristallisieren und/oder Destillieren.

Es ist jedoch auch möglich, die erhaltene Verbindung der Formel II als Rohprodukt ohne weitere Aufreinigung in der folgenden Kondensations- oder Additionsreaktion zur Herstellung der Verbindung der Formel I einzusetzen.

Verbindungen der Formel III sind zum Teil bekannt; ihre Herstellung kann beispielsweise gemäß Angew. Chem. 104, 1652-1654 (1992) oder in Analogie zu dem dort beschriebenen Verfahren erfolgen. Verbindungen der Formel IV sind bekannt und zum Teil kommerziell erhältlich.

Die Kondensation bzw. Addition von Verbindungen der Formel II zu solchen der Formel I kann in an sich bekannter Weise durch Umsetzung mit entsprechenden bifunktionalen Reaktionspartnern erfolgen. Wichtige Reaktionspartner sind beispielsweise Dicarbonsäuren oder Dichloride, Anhydride oder Diester von Dicarbonsäuren; Diisocyanate; Bis-Chlorameisensäureester vom Typ Cl-CO-O-R⁶-O-CO-Cl; Dihalogenverbindungen; Phosgen; Niederalkyl-Kohlensäurediester; Dichlorsilane oder Dichlorsiloxane. Die Erfindung betrifft daher generell auch solche Verbindungen, welche durch Polykondensation oder Polyaddition von Verbindungen der Formel II mit den genannten bifunktionalen Reaktionspartnern erhalten werden können.

Zur Herstellung von Verbindungen der Formel I, worin Z die Bedeutung NH oder NR¹ hat, geht man vielfach von Aminen der Formel VI aus, 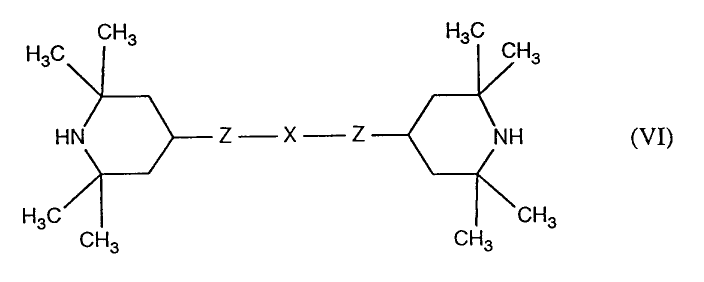 worin Z NH oder NR¹ ist; und X und R¹ die weiter oben angegebenen Bedeutungen haben.

Verbindungen der Formel VI können mittels bekannter Herstellungsverfahren oder in Analogie zu solchen erhalten werden, wie sie beispielsweise in den Publikationen EP-A-388 535; J. Org. Chem. 37, 2015-18 (1992); JP-A-61-241 335 und JP-B-47-007 380 beschrieben sind.

Die Kondensation bzw. Addition von Verbindungen der Formel VI zu solchen der Formel I kann durch Umsetzung mit einem Ester einer aromatischen Sulfonsäure der Formel III bewirkt werden: 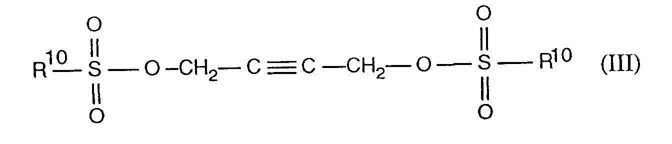
R¹⁰ umfaßt darin die weiter oben angegebenen Bedeutungen. Die Reaktionsbedingungen entsprechen dabei zweckmäßig den weiter oben beschriebenen Reaktionsbedingungen bei der Herstellung der Verbindung der Formel II. Meist werden etwa äquimolare Mengen der beiden Edukte der Formeln VI und III eingesetzt.

Im einzelnen können beispielsweise folgende Klassen von Verbindungen der Formel I erhalten werden:
1. Polyester durch Umsetzung eines Diols der Formel II, worin Z für O steht, mit einer Dicarbonsäure, einem Dichlorid oder Diester einer Dicarbonsäure der Formel E-CO-R²-CO-E, worin E Wasserstoff, Cl oder Niederalkoxy wie z.B. C₁-C₄-Alkoxy ist, oder einem entsprechenden Anhydrid einer Dicarbonsäure. Die Reaktion kann beispielsweise in Analogie zu einer der Reaktionen durchgeführt werden, wie sie in den Methoden der Organischen Chemie (Houben-Weyl), Band E20, Makromolekulare Stoffe, Thieme Verlag, Stuttgart 1987, Seiten 1405 und folgende, besonders Seiten 1409 und 1411-1413 beschrieben sind.
2. Polyurethane beispielsweise durch Umsetzung eines Diols der Formel II, worin Z für O steht, mit einem Diisocyanat der Formel OCN-R⁵-NCO in Analogie zu einem der Verfahren, wie sie in der oben genannten Publikation Methoden der Organischen Chemie (Houben-Weyl), Band E20, Seiten 1587-1703 beschrieben sind. Polyurethane sind weiterhin unter anderem durch Umsetzung eines Diamins der Formel II, worin Z für NH oder NR¹ steht, besonders für NH, mit einem Bis-Chlorameisensäureester vom Typ Cl-CO-O-R⁶-O-CO-Cl erhältlich, wobei entsprechend Methoden der Organischen Chemie (Houben-Weyl), Band E20, Seiten 1710-1715 verfahren werden kann.
3. Polyamide durch Umsetzung von zunächst etwa 2 Äquivalenten einer Verbindung der Formel IV, 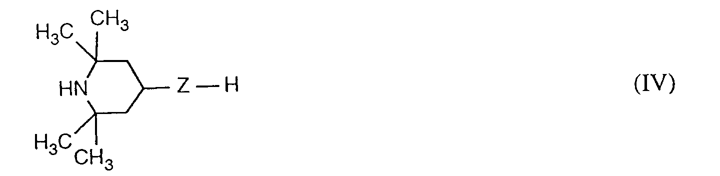 worin Z NH oder NR¹ ist, mit einem Äquivalent einer Dicarbonsäure, einem Dichlorid oder Diester einer Dicarbonsäure der Formel E-CO-R²-CO-E oder E-CO-CO-E, worin E Wasserstoff, Cl oder Niederalkoxy ist, oder einem entsprechenden Anhydrid einer Dicarbonsäure, wobei gemäß einer der in US-A-3 684 765 beschriebenen Methoden vorgegangen werden kann. Das erhaltene Zwischenprodukt der Formel 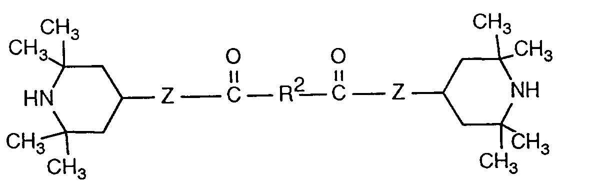 wird anschließend zweckmäßig mit einem Äquivalent der oben beschriebenen Verbindung der Formel III 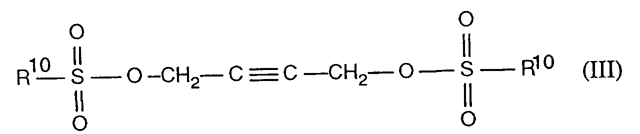 zur Verbindung der Formel I polykondensiert; die Reaktionsbedingungen entsprechen dabei im wesentlichen den oben bereits beschriebenen Bedingungen bei der Umsetzung der Verbindungen der Formeln III und IV zur Verbindung der Formel II.
4. Polyether durch Umsetzung eines Diols der Formel II bzw. des entsprechenden Dialkoholates mit einer Dihalogenverbindung wie z.B. einem Dichlorid Cl-R⁷-Cl, in Analogie zu Verfahren, wie sie beispielsweise in Methoden der Organischen Chemie (Houben-Weyl), 4. Auflage, Band XIV/2, Makromolekulare Stoffe Teil 2, Thieme Verlag, Stuttgart 1963, auf den Seiten 582-583 beschrieben sind.
5. Polycarbonate durch Umsetzung eines Diols der Formel II, worin Z für O steht, unter anderem mit Phosgen, Bis-Chlorameisensäureester von Typ Cl-CO-O-R⁶-O-CO-Cl, Kohlensäure-diestern oder Harnstoff. Die Umsetzung kann beispielsweise in Analogie zu einer der Reaktionen durchgeführt werden, wie sie in den Methoden der Organischen Chemie (Houben-Weyl), Band E20, Makromolekulare Stoffe, Thieme Verlag, Stuttgart 1987, Seiten 1446-1449 beschrieben sind.
6. Polyamine durch Umsetzung eines Amins der Formel VI, worin Z für NR¹ und X für R⁷ steht, mit einer Verbindung der Formel III 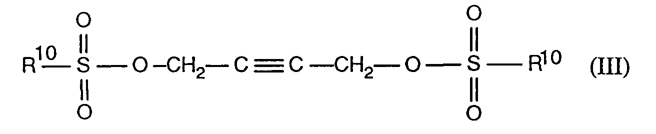
7. Polyharnstoffe durch Umsetzung eines Amins der Formel VI, worin Z für NH oder NR¹ steht und X -CO- oder -CO-NH-R⁵-NH-CO- bedeutet, mit einer Verbindung der Formel III.
8. Polysilane bzw. Polysiloxane durch Umsetzung eines Diols der Formel II bzw. des entsprechenden Dialkoholates mit einer Verbindung der Formel 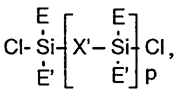 worin E, E', X' und p die oben angegebenen Bedeutungen haben.

Die erfindungsgemäßen Verbindungen lassen sich vorteilhaft zur Stabilisierung organischen Materials gegen den schädigenden Einfluß von Licht, Sauerstoff und/oder Wärme einsetzen. Sie zeichnen sich durch hohe Substratkompatibilität und gute Persistenz im Substrat aus.

Beispiele für erfindungsgemäß zu stabilisierende Materialien sind:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen hoher Dichte und hoher Molmasse (HDPE-HMW), Polyethylen hoher Dichte und ultrahoher Molmasse (HDPE-UHMW), Polyethylen mittlerer Dichte (MDPE), Polyethylen niederer Dichte (LDPE), lineares Polyethylen niederer Dichte (LLDPE), verzweigtes Polyethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metällocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes und bromiertes Copolymer aus Isobutylen-Isopren (Halobutylkautschuk), chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide, Polyetherimide, Polyesterimide, Polyhydantoine und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Harnstoffharzen, Isocyanaten, Isocyanuraten, Polyisocyanaten oder Epoxidharzen vernetzt sind.
26. Vernetzte Epoxidharze, die sich von aliphatischen, cycloaliphatischen, heterocyclischen oder aromatischen Glycidylverbindungen ableiten, z.B. Produkte von Bisphenol-A-diglycidylethern, Bisphenol-F-diglycidylethern, die mittels üblichen Härtern wie z.B. Anhydriden oder Aminen mit oder ohne Beschleunigern vernetzt werden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO, PBT/PC/ABS oder PBT/PET/PC.

Weitere Gegenstände der Erfindung sind daher Zusammensetzungen enthaltend
A) ein gegen oxidativen, thermischen oder/und aktinischen Abbau empfindliches organisches Material und
B) mindestens eine Verbindung der Formel I, sowie die Verwendung von Verbindungen der Formel I zum Stabilisieren von organischen, Material gegen oxidativen, thermischen oder aktinischen Abbau.
Die Erfindung umfaßt ebenfalls ein Verfahren zum Stabilisieren von organischem Material gegen thermischen, oxidativen oder/und aktinischen Abbau, dadurch gekennzeichnet, daß man diesem Material mindestens eine Verbindung der Formel I zusetzt.

Von besonderem Interesse ist die Verwendung von Verbindungen der Formel I als Stabilisatoren in synthetischen organischen Polymeren sowie entsprechende Zusammensetzungen.

Vorzugsweise handelt es sich bei den zu schützenden organischen Materialien um natürliche, halbsynthetische oder bevorzugt synthetische organische Materialien. Besonders bevorzugt sind synthetische organische Polymere oder Gemische solcher Polymere, insbesondere thermoplastische Polymere wie Polyolefine, vor allem Polyethylen und Polypropylen (PP), und Überzugszusammensetzungen.

Als Bindemittel (Komponente A) für die erfindungsgemäßen Überzugszusammensetzungen kommen prinzipiell alle in der Technik gebräuchlichen in Betracht, beispielsweise solche, wie sie beschrieben sind in Ullmann's Encyclopedia of Industrial Chemistry, 5. Ed., Vol. A18, pp. 368-426, VCH, Weinheim 1991. Allgemein handelt es sich um ein filmbildendes Bindemittel basierend auf einem thermoplastischen oder thermohärtbaren Harz, vorwiegend auf einem thermohärtbaren Harz. Beispiele hierfür sind Alkyd-, Acryl-, Polyester-, Phenol-, Melamin-, Epoxid-, Polyurethanharze und deren Gemische.

Das zu stabilisierende organische Material kann auch ein photographisches Material sein; darunter sind insbesondere die Materialien zu verstehen, die in Research Disclosure 1990, 31429 (Seiten 474-480) für die photographische Reproduktion und andere Reproduktionstechniken beschrieben sind.

Allgemein werden die Verbindungen der Formel I dem zu stabilisierenden Material in Mengen von 0,01 bis 10 %, bevorzugt 0,01 bis 5 %, insbesondere 0,01 bis 2 %, zugesetzt (bezogen auf das zu stabilisierende Material). Besonders bevorzugt ist der Einsatz der erfindungsgemäßen Verbindungen in Mengen von 0,05 bis 1,5 %, vor allem 0,1 bis 0,5 %.

Die Einarbeitung in die Materialien kann beispielsweise durch Einmischen oder Aufbringen der Verbindungen der Formel I und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindung auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der Verbindungen der Formel I in Polymere besteht in deren Zugabe vor, wahrend oder unmittelbar nach der Polymerisation der entsprechenden Monomeren bzw. vor der Vernetzung. Die Verbindung der Formel I kann dabei als solche, aber auch in encapsulierter Form (z.B in Wachsen, Ölen oder Polymeren) zugesetzt werden. Im Falle der Zugabe vor oder während der Polymerisation können die Verbindungen der Formel I auch als Regulator für die Kettenlänge der Polymere (Kettenabbrecher) wirken.

Die Verbindungen der Formel I können auch in Form eines Masterbatches, der diese Verbindung beispielsweise in einer Konzentration von 2,5 bis 25 Gew.-% enthält, den zu stabilisierenden Kunststoffen zugesetzt werden.

Zweckmäßig kann die Einarbeitung der Verbindungen der Formel I nach folgenden Methoden erfolgen:
- als Emulsion oder Dispersion (z.B. zu Latices oder Emulsionspolymeren),
- als Trockenmischung während des Vermischens von Zusatzkomponenten oder Polymermischungen,
- durch direktes Zugeben in die Verarbeitungsapparatur (z.B. Extruder, Innenmischer usw.),
- als Lösung oder Schmelze.

Erfindungsgemäße Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Neben den Verbindungen der Formel I können die erfindungsgemäßen Zusammensetzungen als zusätzliche Komponente C ein oder mehrere herkömmliche Additive enthalten, wie beispielsweise die unten angegebenen.

Die erfindungsgemäße Überzugszusammensetzung enthält vorzugsweise neben den Komponenten A und B als Komponente C ein weiteres Lichtschutzmittel vom Typ der sterisch gehinderten Amine, der 2-(2-Hydroxyphenyl)-1,3,5-triazine und/oder der 2-Hydroxyphenyl-2H-benztriazole, beispielsweise wie sie in der folgenden Liste unter den Punkten 2.1, 2.6 und 2.8 aufgeführt sind.

Die herkömmlichen Additive werden zweckmäßig in Mengen von 0,1-10, beispielsweise 0,2-5 Gew.-%, bezogen auf das zu stabilisierende Polymer, eingesetzt.

### 1. Antioxidantien

1.1. Alkylierte Monophenole, z.B. 2,6-Di-tert-butyl-4-methylphenol, 2-Butyl-4,6-di-methylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z.B. 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.
1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis(3,5-di-tert-butyl-4-hydroxyphenyl)adipat.
1.4. Tocopherole, z.B. α-Tocopherol, β-Tocopherol, γ-Tocopherol, δ-Tocopherol und Mischungen davon (Vitamin E).
1.5. Hydroxylierte Thiodiphenylether, z.B. 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis(6-tert-butyl-2-methylphenol), 4,4'-Thio-bis(3,6-di-sec.-amylphenol), 4,4'-Bis(2,6-dimethyl-4-hydroxyphenyl)-disulfid.
1.6. Alkyliden-Bisphenole, z.B. 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis-(4,6-di-tert-butylphenol), 2,2'-Ethyliden-bis(6-tert-butyl-4-isobutylphenol), 2,2'-Methylen-bis[6-(α-methylbenzyl)-4-nonylphenol], 2,2'-Methylen-bis[6-(α,α-dimethylbenzyl)-4-nonylphenol], 4,4' -Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan 2,6-Bis(3-tert-butyl-5-methyl-2-hydroxybenzyl)-4-methylphenol, 1,1,3-Tris(5-tert-butyl-4-hydroxy-2-methylphenyl)-butan, 1,1-Bis(5-tert-butyl-4-hydroxy-2-methyl-phenyl)-3-n-dodecylmercaptobutan, Ethylenglycol-bis[3,3-bis(3'-tert-butyl-4'-hydroxyphenyl)-butyrat], Bis(3-tert-butyl-4-hydroxy-5-methyl-phenyl)-dicyclopentadien, Bis[2-(3'-tert-butyl-2'-hydroxy-5'-methyl-benzyl)-6-tert-butyl-4-methyl-phenyl]-terephthalat, 1,1-Bis(3,5-dimethyl-2-hydroxyphenyl)-butan, 2,2-Bis(3,5-di-tert-butyl-4-hydroxyphenyl)-propan, 2,2-Bis(5-tert-butyl-4-hydroxy-2-methylphenyl)-4-n-dodecylmercapto-butan, 1,1,5,5-Tetra-(5-tert-butyl-4-hydroxy-2-methylphenyl)-pentan.
1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tridecyl-4-hydroxy-3,5-di-tert-butylbenzyl-mercaptoacetat, Tris(3,5-di-tert-butyl-4-hydroxybenzyl)-amin, Bis(4-tert-butyl-3-hydroxy-2,6-dimethylbenzyl)-dithioterphthalat, Bis(3,5-di-tert-butyl-4-hydroxybenzyl)-sulfid, Isooctyl-3,5-di-tert-butyl-4-hydroxybenzyl-mercaptoacetat.
1.8. Hydroxybenzylierte Malonate, z.B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Didodecylmercaptoethyl-2,2-bis(3,5-di-tert-butyl-4-hydroxybenzyl)-malonat, Di-[4-(1,1,3,3-tetramethylbutyl)-phenyl]-2,2-bis(3,5-di-tert-butyl-4-hydroxybenzyl)-malonat.
1.9. Hydroxybenzyl-Aromaten, z.B. 1,3,5-Tris(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6-Tris(3,5-di-tert-butyl-4-hydroxybenzyl)-phenol.
1.10. Triazinverbindungen, z.B. 2,4-Bis-octylmercapto-6-(3,5-di-tert-butyl-4-hydroxyanilino)-1,3,5-thazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris(3,5-di-tert-butyl-4-hydroxybenzyl)-isocyanurat, 1,3,5-Tris(4-tert-butyl-hydroxy-2,6-dimethylbenzyl)-isocyanurat, 2,4,6-Tris(3,5-di-tert-butyl-4-hydroxyphenylethyl)-1,3,5-triazin, 1,3,5-Tris(3,5-di-tert-butyl-4-hydroxyphenylpropionyl)-hexahydro-1,3,5-triazin, 1,3,5-Tris(3,5-dicyclohexyl-4-hydroxybenzyl)-isocyanurat.
1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4 hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
1.13. Ester der β-(3,5-Di-tert-butyl-4-hydroxyphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.14. Ester der β-(5-tert-Butyl-4-hydroxy-3-methylphenyl)-Propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-thoxabicyclo-[2.2.2]-octan.
1.15. Ester der β-(3,5-Dicyclohexyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol; Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-thoxabicyclo-[2.2.2]-octan.
1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.17. Amide der β-(3,5-Di-tert-butyl-4-hydroxyphenyl)-propionsäure, wie z.B. N,N'-Bis(3,5-di-tert-butyl-4-hydroxyphenylpropionyl)-hexamethylendiamin, N,N'-Bis(3,5-di-tert-butyl-4-hydroxyphenylpropionyl)-trimethylendiamin, N,N'-Bis(3,5-di-tert-butyl-4-hydroxyphenylpropionyl)-hydrazin.
1.18. Ascorbinsäure (Vitamin C).
1.19. Aminische Antioxidantien, wie z.B. N,N'-Di-isopropyl-p-phenylendiamin, N,N'-Di-sec-butyl-p-phenylendianlin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N' phenyl-p-phenylendiamin, 4-(p-Toluol-sulfonamido)-diphenyl-amin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tert-octyldiphenylamin, 4-n-Butylaininophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylanlinen, Gemisch aus mono- und dialkylierten Dodecyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.
2. UV-Absorber und Lichtschutzmittel
2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hy-droxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl- 2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phe-nyl)-benzotriazol, 2-(3'-tert-Butyl-5'-[2(2-ethylhexyloxy)carbonylethyl]-2'-hydroxyphenyl)-benzothazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; [R-CH₂CH₂-COO(CH₂)₃]₂ mit R = 3'-tert-Butyl-4'-hydroxy-5'-2H-benzo-triazol-2-yl-phenyl.
2.2. 2-Hydroxybenzophenone, wie z.B. das 4-Hydroxy-, 4-Methoxy-, 4-Octoxy-, 4-Decyl-oxy-, 4-Dodecyloxy-, 4-Benzyloxy-, 4,2',4'-Trihydroxy-, 2'-Hydroxy-4,4'-dimethoxy-Derivat.
2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis(4-tert-butyl-benzoyl)-resorcin, Benzoylresorcin, 3,5-Di-tert-butyl-4-hydroxybenzoesäure-2,4-di-tert butylphenylester, 3,5-Di-tert-butyl-4-hydroxybenzoesäurehexadecylester` 3,5-Di-tert-butyl-4-hydroxybenzoesäure-octadecylester, 3,5-Di-tert-butyl-4-hydroxybenzoesäure-2-methyl-4,6-di-tert-butylphenylester.
2.4. Acrylate, wie z.B. α-Cyan-β,β-diphenylacrylsäure-ethylester bzw. -isooctylester, aXCarbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
2.5. Nickelverbindungen, wie z.B. Nickelkomplexe des 2,2'-Thio-bis[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
2.6. Sterisch gehinderte Amine, wie z.B. Bis(2,2,6,6-tetramethyl-pipendin-4-yl)-sebacat, Bis(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4-yl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethy-4-piperidyl)-1,2,3,4-butantetraoat, 1,1'-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis(1-octyloxy-2,2,6,6-tetramethylpiperidyl)-sebacat, Bis(1-octyloxy-2,2,6,6-tetramethylpiperidyl)-succinat, Kondensationsprodukt aus N,N'-Bis(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylamino)-ethan, Kondensationsprodukt aus 2-Chlor-4,6-di-(4-n-butylamino-1,2,2,6,6-pentanethylpiperidyl)-1,3,5-triazin und 1,2-Bis(3-aminopropylamino)-äthan, 8-Acetyl-3-dodecyl-7,7,9,9-tetramethyl-1,3,8-triazaspiro[4,5]decan-2,4-dion, 3-Dodecyl-1-(2,2,6,6-tetramethyl-4-piperidyl)pyrrolidin-2,5-dion, 3-Dodecyl-1-(1,2,2.6,6-pentamethyl-4-piperidyl)-pyrrolidin-2,5-dion, Gemisch von 4-Hexadecyloxy- und 4-Stearyloxy-2,2,6,6-tetramethylpiperidin, Kondenstionsprodukt aus N,N'-Bis(2,2,6,6-tetramethyl-4-piperidyl)-hexamethylendiamin und 4-Cyclohexylamino-2`6-dichlor-1,3,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reh. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan, Umsetzungsprodukt von 7,7,9,9-Tetramethyl-2-cycloundecyl-1-oxa-3,8-diaza-4-oxospiro[4,5]decan und Epichlorhydrin.
2.7. Oxalsäurediamide, wie z.B. 4,4'-Di-octyloxy-oxanilid, 2,2'-Diethoxy-oxanilid. 2,2'-Di-octyloxy-5`5'-di-tert-butyl-oxanilid` 2,2'-Di-dodecyloxy-5,5'-di-tert-butyl-oxanilid, 2-Ethoxy-2'-ethyl-oxanilid, N,N'-Bis(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-subslituierten Oxaniliden.
2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis-(2,4-dimethylphenyl)-1,3,5-triazin, 2-[2-Hydroxy-4-(2-hydroxy-3-butyloxy-propyloxy)-phenyl]-4,6-bis(2,4-dimethylphenyl)-1,3,5-triazin, 2-[2-Hydroxy-4-(2-hydroxy-3-octyl-oxy-propyloxy)pheny]-4,6-bis(2,4-dimethylphenyl)-1,3,5-thazin, 2[4-(dodecyloxy/tridecyloxy-2-hydroxypropoxy)-2-hydroxy-phenyl]-4,6-bis(2,4-dimethylphenyl)-1,3,5-thazin, 2-[2-Hydroxy-4-(2-hydroxy-3-dodecyloxy-propoxy)phenyl]-4,6-bis(2,4-dimethylphenyl)-1,3,5-triazin, 2-(2-Hydroxy-4-hexyloxy)phenyl-4,6-diphenyl-1,3,5-triazin, 2-(2-Hydroxy-4-methoxyphenyl)-4,6-diphenyl 1,3,5-triazin, 2,4,6-Tris[2-hydroxy-4-(3-butoxy-2-hydroxy-propoxy)phenyl]- 1,3,5-thazin, 2-(2-Hydroxyphenyl)-4-(4-methoxyphenyl)-6-phenyl-1,3,5-triazin.
3. Metalldesaktivatoren, wie z.B. N,N'-Diphenyloxalsäurediamid, N-Salicylal-N'-salicyloylhydrazin, N,N'-Bis(salicyloyl)-hydrazin, N,N'-Bis(3,5-di-tert-butyl-4-hydroxyphenyl-propionyl)-hydrazin, 3-Salicyloylamino- 1,2,4-triazol, Bis(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Phosphite und Phosphonite, wie z.B. Triphenylphosphit, Diphenylalkylphosphite, Phenyldialkylphosphite, Tris(nonylphenyl)-phosphit, Trilaurylphosphit, Trioctadecylphosphit, Distearyl-pentaerythritdiphosphit, Tris(2,4-di-tert-butylphenyl)-phosphit, Diisodecylpentaerythrit-diphosphit, Bis(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis(2,4-di-tert-butyl-6-methylphenyl)-pentderythritdiphosphit, Bis-(2,4,6-tri-tert-butylphenyl)-pentaerythritdiphosphit, Tristearyl-sorbit-triphosphit, Tetrakis(2,4-di-tert-butylphenyl)-4,4'-biphenylen-diphosphonit, 6-Isooctyloxy-2,4,8,10-tetra-tert-butyl-12H-di-benz[d,g]-1,3,2-dioxaphosphocin, 6-Fluor-2,4,8,10-tetra-tert-butyl-12-methyl-dibenz-[d,g]-1,3,2-dioxaphosphocin, Bis(2,4-di-tert-butyl-6-methylphenyl)-methylphosphit, Bis(2,4-di-tert-butyl-6-methylphenyl)-elhylphosphit.
5. Hydroxylamine wie z.B. N,N-Dibenzylhydroxylamin, N,N-diethylhydioxylamin, N,N-Dioctylhydroxylamin, N,N-Dilaurylhydroxylamin, N,N-Ditetradecylhydroxylamin N,N-Dihexadecylhydroxylamin, N,N-Dioctadecylhydroxylamin, N-Hexadecyl-N-octadecylhydroxylamin, N-Heptadecyl-N-octadecylhydroxylamin, N,N-Dialkylhydroxylamin aus hydrierten Talgfettaminen.
6. Nitrone wie z.B. N-Benzyl-alpha-phenyl-nitron, N-Ethyl-alpha-methyl-nitron, N-Octyl-alpha-heptyl-nitron, N-Lauryl-alpha-undecyl-nitron, N-Tetradecyl-alpha-tridecylnitron, N-Hexadecyl-alpha-pentadecyl-nitron, N-Octadecyl-alpha-heptadecyl-nitron, N-Hexadecyl-alpha-heptadecyl-nitron, N-Ocatadecyl-alpha-pentadecyl-nitron, N-Heptadecyl-alpha-heptadecyl-nitron, N-Octadecyl-alpha-hexadecyl-nitron, Nitrone abgeleitet von N,N-Dialkylhydroxylaminen hergestellt aus hydrierten Talgfettaminen.
7. Thiosynergisten wie z.B. Thiodipropionsäure-di-laurylester oder Thiodipropionsäuredi-stearylester.
8. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis(β-dodecylmercapto)-propionat.
9. Polyamidstabilisatoren, wie z.B. Kupfersalze in Kombination mit Jodiden und/oder Phosphorverbindungen und Salze des zweiwertigen Mangans.
10. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
11. Nukleierungsmittel, wie z.B. anorganische Stoffe wie z.B. Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z.B. 4-tert-Butylbenzoesäure, Adipinsäure, Diphenylessigsäure, Natriumsuccinat oder Natriumbenzoat; polymere Verbindungen wie z.B. ionische Copolymerisate ("Ionomere").
12. Füllstoffe und Verstärkungsmittel, wie z.B. Calciumcarbonat, Silikate, Glasfasern, Glaskugeln, Asbest, Talk, Kaolin, Glimmer, Bariumsulfat, Metalloxide und -hydroxide, Ruß, Graphit, Holzmehl und Mehle oder Fasern anderer Naturprodukte, synthetische Fasern.
13. Sonstige Zusätze, wie z.B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.
14. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis[5,7-di-tertbutyl-3-(4-[2-hydroxyethoxy]-phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Die nachfolgenden Beispiele illustrieren die Erfindung weiter. Alle Angaben in Teilen oder Prozenten, in den Beispielen ebenso wie in der übrigen Beschreibung sowie in den Patentansprüchen, beziehen sich auf das Gewicht, sofern nichts anderes angegeben ist. Die berechneten Analysendaten der polymeren Verbindungen sind auf die wiederkehrende Einheit bezogen (gleichbedeutend mit unendlicher Kettenlänge).
In den Beispielen werden die folgenden Abkürzungen verwendet:
- GC:: Gaschromatographie;
- HPLC: Hochdruck-Flüssigchromatographie;
- GPC:: Gelpermeationschromatographie;
- THF:: Tetrahydrofuran;
- MALDI:: Matrix Assisted Laser Desorption Ionization;
- MS:: Massenspektrometrie;
- DSC:: Differential-Thermoanalyse;
- Mₙ:: Zahlenmittel der Molmasse (Einheit g/Mol);
- M_{w}:: Massenmittel der Molmasse (Einheit g/Mol);
- H-NMR:: Magnet. Kernresonanz des Nuklids ¹H.
1 Torr entspricht einem Druck von ca. 133 Pa.

### A) Herstellungsbeispiele

### A1) 2-Butin-1,4-diol-ditosylat (Verbindung 1) wird hergestellt gemäß Angew. Chem. 104, 1652-1654 (1992).

### A2) Herstellung von 1,4-Bis-(4-Hydroxy-2,2,6,6-tetramethylpiperidinyl)-but-2-in

In einem Rundkolben mit Magnetrührer und Kühler werden unter Argon 833 g (5,3 Mol) 4-Hydroxy-2,2,6,6-tetramethylpiperidin und 418 g (1,05 Mol) 2-Butin-1,4-diol-ditosylat (Verbindung 1) in 3,6 1 Acetonitril vorgelegt.
Die Mischung wird über Nacht am Rückfluss gekocht.
Dann wird auf Eis gegossen und mit Essigester extrahiert. Die organische Phase wird getrocknet und eingedampft.
Das Rohprodukt wird in Ethanol umkristallisiert. Man erhält 295 g (76 %) des Titelproduktes der Formel 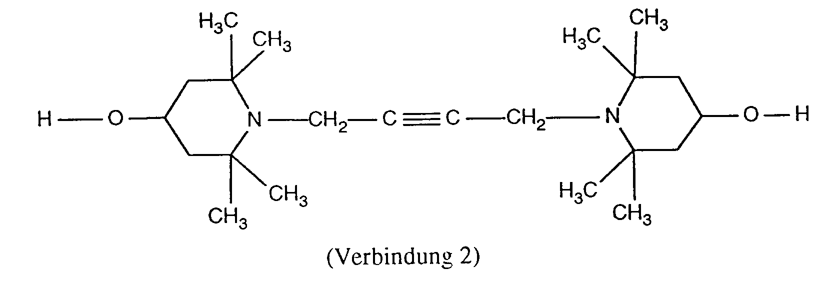 als weisse Kristalle von, Schmelzpunkt 203,4°C.

| Mikroanalyse | berechnet | gefunden |
|---|---|---|
| C | 72,48 | 72,52 |
| H | 11,06 | 11,12 |
| N | 7,68 | 7,75 |

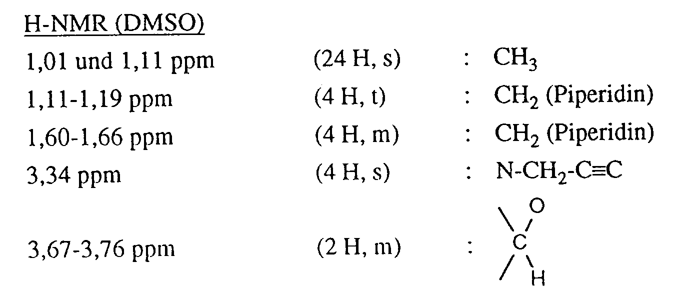

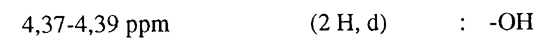

### A3) Polykondensation von Verbindung 2 mit Sebacinsäuredimethylester

### 3a) Diol: Diester= 1:1

In einem 3 Hals Rundkolben mit Magnetrührer, Destillationsaufsatz und Argoneinlaß werden 16,91 g (73,4 mmol) Sebacinsäuredimethylester, 26,77 g (73,4 mmol) der Verbindung 2 und 2 g Dibutylzinnoxid in 300 ml Xylol gelöst. Es wird auf 150°C erwärmt und ein leichter Algonstrom wird durch die Reaktionslösung geblasen.
Nach 20 Std. wird Xylol bei 20 Torr (2666 Pa) abdestilliert, dann wird am Hochvakuum getrocknet.
Der Rückstand wird in 150 ml Toluol gelöst und in Acetonitril gegeben, wobei das Polymer ausfällt. Diese Operation wird wiederholt.
Der Polyester wird am Hochvakuum bei 25°C getrocknet. Man erhält 37 g (95 %) weißes Polymerpulver.
- GPC (THF): Mₙ = 6300
M_{w} = 13400

| Mikroanalyse | ber. | gef. |
|---|---|---|
| C | 72,41 | 70,20 |
| H | 10,25 | 10,13 |
| N | 5,28 | 4,74 |

### 3b) Diol: Diester = 10:9

Analog 3a) werden 20,7 g (90 mmol) Sebacinsäuredimethylester mit 36,5 g (0,1 Mol) Verb. 2 polykondensiert.
Man erhält 29g (61 %) Polymer
- GPC (THF): Mₙ = 5400
M_{w} = 10600

### 3c) Diol: Diester = 10:8

Analog 3a) werden 18,4 g (80 mmol) Sebacinsäuredimethylester mit 36,5 g (0,1 Mol) Verb. 2 polykondensiert.
Man erhält 22 g (52 %) Polymer
- GPC (THF): Mₙ = 3500
M_{w} = 6600

### A4) Polykondensation von Verbindung 2 mit Hexamethylendiisocyanat

In einem 500 ml Rundkolben mit Kühler und Magnetrührer werden unter Argon werden 36,5 g (0,1 Mol) Verb. 2 und 16,8 g (0,1 Mol) Hexamethylendiisocyanat in 200 ml wasserfreiem Chlorbenzol gelöst und 8 Std. am Rückfluß gekocht. Die Lösung wird auf Acetonitril gegossen, wobei das Polymer ausfällt. Dieses wird abgetrennt, in Chloroform gelöst und erneut in Acetonitril gefällt. Das weiße Polymer wird am Hochvakuum bei 50°C getrocknet. Ausbeute 46 g (86 %) Mikroanalyse

| | berechnet | gefunden |
|---|---|---|
| C | 67,63 | 66,80 |
| H | 9,84 | 9,98 |
| N | 10,52 | 10,28 |

- GPC (THF): Mₙ = 18000
M_{w} = 25000

### A5) Polykondensation von Verbindung 2 mit Bernsteinsäuredimethylester

### 5a) Diol: Diester = 1:1

In einem 3 Hals Rundkolben mit Magnetrührer, Thermometer, Argoneinlaß und Destillationsaufsatz werden 36,5 g (0,1 Mol) Verbindung 2, 14,6 g (0,1 Mol) Bernsteinsäuredimethylester und 2,7 g Dibutylzinnoxid in 400 ml Xylol gelöst. Man erwärmt die Lösung auf 145°C und leitet einen leichten Argonstrom durch die Lösung.
Nach dem vollständigen Abdestillieren des Methanols wird die Temperatur so weit erhöht, daß das Lösungsmittel abdestilliert.
Der Rückstand wird in THF gelöst und in Acetonitril gefällt Dieser Vorgang wird nochmals wiederholt und das Polymer bei 40°C/0,01 Torr getrocknet; Ausbeute 33 g (74%)

| Mikroanalyse | | |
|---|---|---|
| | berechnet | gefunden |
| C | 69,92 | 67,67 |
| H | 9,48 | 9,51 |
| N | 6,27 | 6,09 |

- GPC (THF): Mₙ = 6000
M_{w} = 12200

### 5b) Diol: Diester = 10:9

Analog 5a) werden 36,5 g (0,1 Mol) Verbindung 2 und 13,2 g (0,09 Mol) Bernsteinsäuredimethylester polykondensiert.
Man erhält 31,5 g (78 %) weißes Polymerpulver.
- GPC (THF): Mₙ = 4600
M_{w} = 8200

### 5c) Diol: Diester = 10:8

Analog 5a) werden 36,5 g (0,1 Mol) Verbindung 2 mit 11,7 g (0,08 Mol) Bernsteinsäuredimethylester umgesetzt. Ausbeute: 19,2 g (54 %)
- GPC (THF): Mₙ = 3300
M_{w} = 5800

### A6) Polykondensation von Verbindung 2 mit Isophthalsäuredimethylester

Analog 5a) werden 36,5 g (0,1 Mol) Verbindung 2 mit 19,4 g (0,1 Mol) Isophthalsäuredimethylester umgesetzt; Ausbeute: 35 g (71 %)

| Mikroanalyse | | |
|---|---|---|
| | berechnet | gefunden |
| C | 72,55 | 70,73 |
| H | 8,93 | 8,63 |
| N | 5,64 | 5,12 |

- GPC (THF): Mₙ = 5000
M_{w} = 12500

### A7) Polykondensation von Verbindung 2 mit Phosgen

In einem 350 ml Sulfierkolben mit mechanischem Rührer, Gaseinleitungsrohr und Innenthermometer werden 36,5 g (0,1 Mol) Verbindung 2 in 230 ml Pyridin aufgenommen.

Bei Einleitung von Phosgen bei 20°C erfolgt eine exotherme Reaktion, während deren Verlauf die Innentemperatur bei 25-35°C gehalten wird. Die Einleitung wird fortgeführt, bis eine viskose Suspension erhalten wird. Anschließend wird mit Stickstoff gespült, in 100 ml Methanol aufgenommen und auf Wasser gegossen. Der weiße Niederschlag wird abgetrennt, mit Wasser gewaschen und getrocknet. Anschließend wird in Dichlormethan gelöst und in der zehnfachen Menge Methanol gefällt. Das weiße Polymer wird bei 30°C am Hochvakuum getrocknet. Ausbeute: 17,1 g (44 %)
- GPC (THF): Mₙ = 5400
M_{w} = 11000

| Mikroanalyse | | |
|---|---|---|
| | berechnet | gefunden |
| C | 70,73 | 69,82 |
| H | 9,81 | 9,71 |
| N | 7,17 | 7,01 |

### A8) Polykondensation von Verbindung 2 mit Dibrommethan

In einem 750 ml Sulfierkolben mit mechanischen, Rührer, Kühler und Innenthermometer werden 112 g Kaliumhydroxid in 40 ml Wasser gelöst. Dazu wird eine Lösung von 36,5 g (0,1 Mol) der Verbindung 2 in 200 ml Chlorbenzol gegeben, gefolgt von 17,4 g (0,1 Mol) Dibrommethan.
Unter Rühren wird auf 60°C erwärmt und 35,5 g (0,11 Mol) Tetrabutylammoniumbromid dazugegeben. Nach Einsetzen einer exothermen Reaktion steigt die Innentemperatur bis 77°C an. Es wird für ca. 15 h bei 60°C weiter gerührt, dann wird auf 20-25°C gekühlt und mit 200 ml Dichlormethan verdünnt. Man gießt auf Eiswasser, trennt die organische Phase ab, wäscht diese mit Kochsalzlösung, trocknet mit Natriumsulfat und trennt das Lösungsmittel am Rotationsverdampfer ab.
Der feste Rückstand wird in n-Hexan für vier Stunden extrahiert, anschließend am Hochvakuum getrocknet. Ausbeute: 23,5 g (62 %).
- GPC (THF):: Mₙ = 935
M_{w} = 1320

| Mikroanalyse: | | |
|---|---|---|
| | berechnet | gefunden |
| C | 73,36 | 70,54 |
| H | 10,71 | 10,64 |
| N | 7,44 | 7,02 |

### A9) Polykondensation von Verbindung 2 mit Diphenyldichlorsilan

In einem 750 ml Sulfierkolben mit Magnetrührer, Tropftrichter, Kühler und Argonballon werden 22,8 g (90 mmol) Diphenyldichlorsilan in 250 ml Dioxan gelöst. Dazu gibt man 36,5 g der Verbindung 2. Zur weißen Suspension werden tropfenweise bei anfangs 20-25°C 21,2 g (210 mmol) Triethylamin in 50 ml Dioxan gegeben, wobei die Temperatur der Reaktionsmischung auf 39°C steigt. Es wird auf 60°C erwärmt und für 15 h gerührt. Anschließend wird auf Eiswasser gegossen, der weiße Feststoff getrocknet, in 300 ml **THF** gelöst und die Lösung auf Acetonitril getropft, wobei das Polymer ausfällt. Das Polymer wird bei 50°C an, Hochvakuum getrocknet. Ausbeute: 36,2 g (74%).

| Analyse | berechnet | gefunden |
|---|---|---|
| % C | 74,95 | 74,28 |
| % H | 8,88 | 8,87 |
| % N | 5,14 | 5,00 |

- MALDI-MS: Mₙ = 3226
M_{w} = 4942

### B) Anwendungsbeispiele

### Beispiel B1: Lichtstabilisierung von Polypropylenfasern

Je 2,5 g des erfindungsgemäßen Stabilisators werden zusammen mit 1 g Tris(2,4-di-tert.-butylphenyl)phosphit, 1 g Calcium-Monoethyl-3,5-di-tert.butyl-4-hydroxybenzylphosphonat, 1 g Calciumstearat und 2,5 g TiO₂ (Kronos RN 57) in einem Turbomischer mit 1000 g Polypropylenpulver (Moplen® FLF 20) vermischt (Schmelzindex 12 g/10 min, gemessen bei 230°C/2,16 kg).
Die Mischungen werden bei 230°C zu Granulat extrudiert; dieses wird anschließend mit Hilfe einer Pilotanlage (Leonard; Sumirago/VA, Italy) unter folgenden Bedingungen zu Fasern verarbeitet:
- Extrudertemperatur:: 230-240-245-260-255-255°C
- Streckverhältnis:: 1:3,5
- Strecktemperatur:: 100°C
- Fasern:: 12 den

Die so hergestellten Fasern werden vor weißem Hintergrund in einem Weather-O-Meter® Typ 65WR (Atlas Corp.) mit einer Schwarztafeltemperatur von 63°C gemäß ASTM D 2565-85 belichtet. Nach unterschiedlichen Belichtungszeiten wird die verbliebene Zugfestigkeit der Proben gemessen. Aus den Meßwerten wird die Belichtungszeit T₅₀ berechnet, nach der nur noch 50 % der ursprünglichen Zugfestigkeit vorhanden ist.

Zu Vergleichszwecken werden Fasern ohne erfindungsgemäßen Stablisator unter sonst gleichen Bedingungen hergestellt und getestet. Die Testergebnisse sind in Tabelle B1 zusammengestellt.

**Tab. B1**

| Belichtungsdauer bis Halbierung der anfänglichen Zugfestigkeit | |
|---|---|
| Stabilisator | Belichtungsdauer |
| keiner | 220 h |
| aus Beispiel A3a | 1620 h |
| aus Beispiel A5a | 1460 h |
| aus Beispiel A6 | 1200 h |

Die erfindungsgemäß stabilisierten Fasern weisen einen hervorragenden Festigkeitserhalt auf.

### Beispiel B2: Lichtstabilisierung von Polypropylenbändern

Je 1,0 g des erfindungsgemäßen Stabilisators werden zusammen mit 0,75 g Tris(2,4-di-tert.butylphenyl)phosphit, 0,75 g Calcium-Monoethyl-3,5-di-tert.butyl-4-hydroxybenzylphosphonat und 1 g Calciumstearat in einem Turbomischer mit 1000 g Polypropylenpulver vermischt (Schmelzindex 4,0 g/10 min, gemessen bei 230°C/2,16 kg). Die Mischungen werden bei 200-230°C zu Granulat extrudiert; dieses wird anschließend mit Hilfe einer Pilotanlage (Leonard; Sumirago/VA, Italy) unter folgenden Bedingungen zu Streckbändern von 50 µm Dicke und 2,5 mm Breite verarbeitet:
- Extrudertemperatur:: 210-230°C
- Kopftemperatur:: 240-260°C
- Streckverhältnis:: 1:6

Die so hergestellten Bänder werden vor weißem Hintergrund in einem Weather-O-Meter® Typ 65WR (Atlas Corp.) mit einer Schwarztafeltemperatur von 63°C gemäß ASTM D 2565-85 belichtet. Nach unterschiedlichen Belichtungszeiten wird die verbliebene Zugfestigkeit der Proben gemessen. Aus den Meßwerten wird die Belichtungszeit T₅₀ berechnet, nach der nur noch 50 % der ursprünglichen Zugfestigkeit vorhanden ist.

Zu Vergleichszwecken werden Fasern ohne erfindungsgemäßen Stablisator unter sonst gleichen Bedingungen hergestellt und getestet. Die Testergebnisse sind in Tabelle B2 zusammengestellt.

**Tab. B2**

| Belichtungsdauer bis Halbierung der anfänglichen Zugfestigkeit | |
|---|---|
| Stabilisator | Belichtungsdauer |
| keiner | 500 h |
| aus Beispiel A3a | 1620 h |
| aus Beispiel A4 | 1840 h |

Die erfindungsgemäß stabilisierten Bänder weisen einen hervorragenden Festigkeitserhalt auf.

## Patentansprüche

1. Verbindung der Formel I 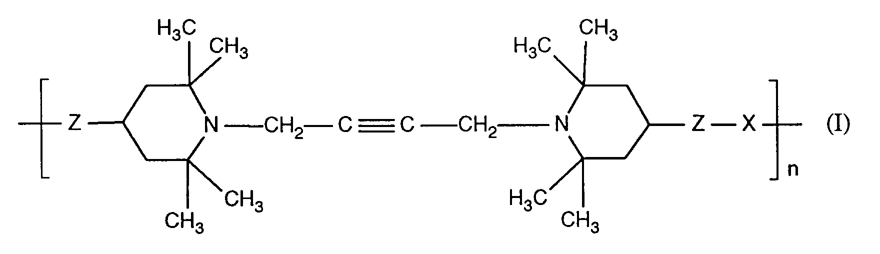
Worin n eine Zahl aus dem Bereich 1 bis 100 ist;
X die Bedeutungen -R⁷-, -CO-, -CO-CO-, -CO-R²-CO-, -CO-NH-R⁵-NH-CO- und -CO-O-R⁶-O-CO- und 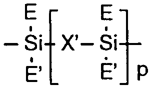 umfaßt, wobei p eine Zahl aus dem Bereich 0-6, X' O oder eine direkte Bindung, E C₁-C₂-Alkyl, Phenyl, C₇-C₁₂-Phenylalkyl oder C₅-C₁₂-Cycloalkyl bedeutet, und E' Wasserstoff ist oder eine der für E gegebenen Bedeutungen hat;
Z im Fall, daß X R⁷ ist, O oder NR¹ ist; Z im Fall, daß 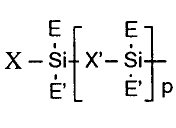 ist, O bedeutet; und
Z in allen anderen Fallen für O, NH oder NR¹ steht;
R¹ C₁-C₈-Alkyl, C₇-C₉-Phenylalkyl, C₅-C₇-Cycloalkyl, C₂-C₈-Alkanoyl, Benzoyl oder durch C₁-C₄-Alkyl substituiertes Benzoyl darstellt;
R² und R⁷ C₁-C₁₈-Alkylen; durch O oder S unterbrochenes C₂-C₁₈-Alkylen; Phenylen; einen gemischt aromatisch/aliphatischen Rest enthaltend 7 bis 12 Kohlenstoffatome; Naphthylen; oder einen Rest -R⁴-D-R⁴- bedeuten;
R⁴ 1,4-Cyclohexylen oder 1,4-Phenylen; und
R⁶ Phenylen, Naphthylen oder 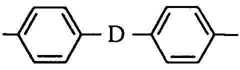 bedeutet;
R⁵ im Fall, daß Z O ist, eine der für R² angegebenen Bedeutungen hat; und
R⁵ im Fall, daß Z NH oder NR¹ ist, eine der für R⁶ angegebenen Bedeutungen hat; und
D C₁-C₃-Alkylen, -S-,-SO-, -SO₂-,-CO-, -O-, -NH- oder -NR¹- darstellt.

2. Verbindung der Formel I gemäß Anspruch 1, worin n eine Zahl aus dem Bereich 4 bis 80 ist.

3. Verbindung der Formel I gemäß Anspruch 1, worin
X die Bedeutungen -CO-, -CO-CO-, -CO-R²-CO-, 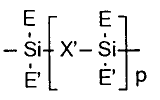 und -CO-NH-R⁵-NH-CO- umfaßt, wobei p 0 oder 1 ist, X' O oder eine direkte Bindung, E' Wasserstoff, Phenyl oder Methyl und E Phenyl oder Methyl darstellt;
R¹ C₁-C₈-Alkyl, Benzyl, C₂-C₈-Alkanoyl, Benzoyl oder Cyclohexyl darstellt;
R² und R⁷ C₁-C₁₈-Alkylen; durch O oder S unterbrochenes C₂-C₁₈-Alkylen; Phenylen; einen gemischt aromatisch/aliphatischen Rest enthaltend 7 bis 12 Kohlenstoffatome; Naphthylen; oder einen Rest -R⁴-D-R⁴-;
R⁴ 1 ,4-Cyclohexylen oder 1,4-Phenylen;
R⁵ im Fall, daß Z O ist, C₃-C₁₂-Alkylen, Phenylen, Naphthylen, Cyclohexylen, Tolylen, durch Phenylen unterbrochenes Ethylen, oder -R⁴-CH₂-R⁴- bedeutet; und
D C₁-C₃-Alkylen, -NH-, -NR¹-, -CO- oder -S- darstellt.

4. Verbindung der Formel I gemäß Anspruch 1, worin
Z für O oder NR¹ steht;
X die Bedeutungen -CO-CO-, -CO-R²-CO-, 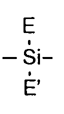 und -CO-NH-R⁵-NH-CO- umfaßt;
R² und R⁷ C₁-C₁₈-Alkylen; Phenylen, CH₂-S-CH₂, CH₂-O-CH₂ oder 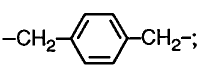 und
R⁵ im Fall, daß Z O ist, C₃-C₁₂-Alkylen, Phenylen, Naphthylen, Cyclohexylen, Tolylen, durch Phenylen unterbrochenes Ethylen, oder -R⁴-CH₂-R⁴- darstellt, wobei R⁴ 1,4-Cyclohexylen oder 1,4-Phenylen ist; und R⁵ im Fall, daß Z NR¹ ist, Phenylen bedeutet.

5. Verbindung der Formel I gemäß Anspruch 4, worin n eine ganze Zahl aus dem Bereich 5 bis 50;
Z ein Sauerstoffatom bedeutet;
R² und R⁷ C₁-C₁₂-Alkylen oder Phenylen; und
R⁵ C₃-C₁₂-Alkylen, Phenylen, Naphthylen oder Cyclohexylen ist.

6. Zusammensetzung enthaltend A) ein gegen oxidativen, thermischen oder/und aktinischen Abbau empfindliches organisches Material und B) mindestens eine Verbindung der Formel I, gemäß Anspruch 1.

7. Zusammensetzung gemäß Anspruch 6 enthaltend als Komponente A ein synthetisches organisches Polymer.

8. Zusammensetzung gemaß Anspruch 6 enthaltend 0,01 bis 10 % der Verbindung der Komponente B, bezogen auf das zu stabilisierende Material.

9. Zusammensetzung gemäß Anspruch 6 enthaltend als zusätzliche Komponente C ein oder mehrere herkömmliche Additive.

10. Verwendung von Verbindungen der Formel I gemäß Anspruch 1 zum Stabilisieren von organischem Material gegen oxidativen, thermischen oder aktinischen Abbau.

11. Verfahren zum Stabilisieren von organischem Material gegen thermischen, oxidativen oder/und aktinischen Abbau, dadurch gekennzeichnet, daß man diesem Material mindestens eine Verbindung der Formel I gemäß Anspruch 1 zusetzt.

12. Verbindung, erhältlich durch Polykondensation oder Polyaddition einer Verbindung der Formel II 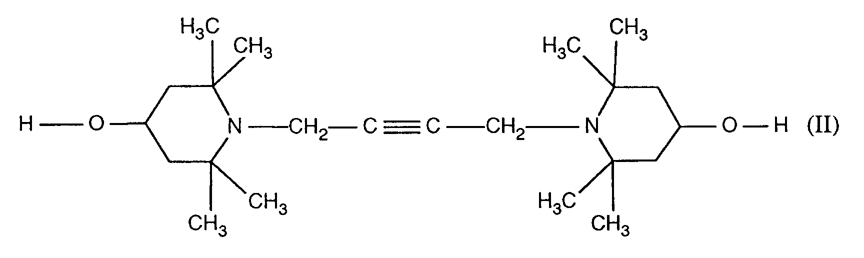 mit einem bifunktionalen Reaktionspartner ausgewählt aus Dicarbonsäuren, Dichloriden oder Diestern von Dicarbonsäuren der Formel G-CO-R²-CO-G oder G-CO-CO-G, worin G Wasserstoff, Cl oder C₁-C₄-Alkoxy ist; oder einem Anhydrid der Formel 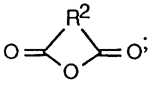 Diisocyanaten der Formel OCN-R⁵-NCO; Dihalogenverbindungen der Formel Hal-R⁷-Hal, worin Hal jeweils für ein Halogenatom steht; Phosgen; C₁-C₄-Alkyl-Kohlensäurediestern; oder Bis-Chlorameisensäureestern vom Typ Cl-CO-O-R⁶-O-CO-Cl, worin
R², R⁵ und R⁷ C₁-C₁₈-Alkylen; durch O oder S unterbrochenes C₂-C₁₈-Alkylen; Phenylen; einen gemischt aromatisch/aliphatischen Rest enthaltend 7 bis 12 Kohlenstoffatome; Naphthylen; oder einen Rest -R⁴-D-R⁴- darstellen;
R⁴ 1,4-Cyclohexylen oder 1,4-Phenylen ist;
R⁶ Phenylen, Naphthylen oder 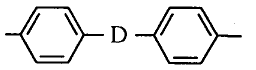 bedeutet; und
D C₁-C₃-Alkylen, -S-,-SO-, -SO₂-,-CO-, -O-, -NH- oder -NR¹- darstellt.

## Claims

1. A compound of the formula I 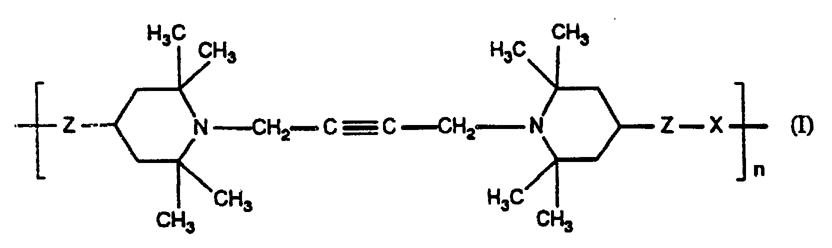
in which n is a number in the range from 1 to 100;
X is -R⁷-, -CO-, -CO-CO-, -CO-R²-CO- -CO-NH-R⁵-NH-CO-, -CO-O-R⁶-O-CO- or 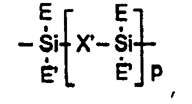 where p is a number in the range from 0 to 6, X' is O or a direct bond, E is C₁-C₁₂alkyl, phenyl, C₇-C₁₂phenylalkyl or C₅-C₁₂cycloalkyl, and E' is hydrogen or is as defined for E;
Z, when X is R⁷, is O or NR¹; Z, when X is 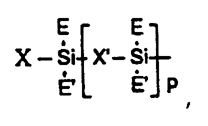 is O; and
Z, in all other cases, is O, NH or NR¹;
R¹ is C₁-C₈alkyl, C₇-C₉phenylalkyl, C₅-C₇cycloalkyl, C₂-C₈alkanoyl, benzoyl or C₁-C₄alkyl-substituted benzoyl;
R² and R⁷ are C₁-C₁₈alkylene; C₂-C₁₈alkylene which is interrupted by O or S; phenylene; a mixed aromatic/aliphatic radical containing 7 to 12 carbon atoms; naphthylene; or an -R⁴-D-R⁴- radical;
R⁴ is 1,4-cyclohexylene or 1,4-phenylene; and
R⁶ is phenylene, naphthylene or 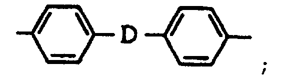
R⁵, when Z is O, is as defined for R²; and
R⁵, when Z is NH or NR¹, is as defined for R⁶; and
D is C₁-C₃alkylene, -S-, -SO-, -SO₂-, -CO-, -O-, -NH- or -NH¹-.

2. A compound of the formula I according to claim 1, in which n is a number in the range from 4 to 80.

3. A compound of the formula I according to claim 1, in which
X is -CO-,-CO-CO-,-CO-R²-CO-, 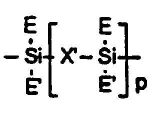 or -CO-NH-R⁵-NH-CO-, where p is 0 or 1, X' is O or a direct bond, E' is hydrogen, phenyl or methyl, and E is phenyl or methyl;
R¹ is C₁-C₈alkyl, benzyl, C₂-C₈alkanoyl, benzoyl or cyclohexyl;
R² and R⁷ are C₁-C₈alkylene; C₂-C₁₈alkylene which is interrupted by O or S; phenylene; a mixed aromatic/aliphatic radical containing 7 to 12 carbon atoms; naphthylene; or an -R⁴-D-R⁴- radical;
R⁴ is 1,4-cyclohexylene or 1,4-phenylene;
R⁵, when Z is O, is C₃-C₁₂alkylene, phenylene, naphthylene, cyclohexylene, tolylene, ethylene which is interrupted by phenylene, or -R⁴-CH₂-R⁴-; and
D is C₁-C₃alkylene, -NH-, -NR¹-, -CO- or -S-.

4. A compound of the formula I according to claim 1, in which
Z is O or NR¹;
X is -CO-CO-,-CO-R²-CO-, 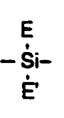 or -CO-NH-R⁵-NH-CO-;
R² and R⁷ are C₁-C₁₈alkylene; phenylene, CH₂-S-CH₂, CH₂-O-CH₂ or 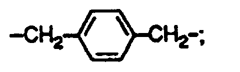 and
R⁵, when Z is O, is C₃-C₁₂alkylene, phenylene, naphthylene, cyclohexylene, tolylene, ethylene which is interrupted by phenylene, or -R⁴-CH₂-R⁴-, where R⁴ is 1,4-cyclohexylene or 1,4-phenylene; and R⁵, when Z is NR¹, is phenylene.

5. A compound of the formula I according to claim 4, in which n is an integer in the range from 5 to 50;
Z is an oxygen atom;
R² and R⁷ are C₁-C₁₂alkylene or phenylene; and
R⁵ is C₃-C₁₂alkylene, phenylene, naphthylene or cyclohexylene.

6. A composition comprising A) an organic material which is sensitive to oxidative, thermal and/or actinic degradation, and
B) at least one compound of the formula I according to claim 1.

7. A composition according to claim 6, wherein component A is a synthetic organic polymer.

8. A composition according to claim 6, comprising from 0.01 to 10 % of the compound of component B, based on the material to be stabilized.

9. A composition according to claim 6, comprising, as an additional component C, one or more conventional additives.

10. The use of a compound of the formula I according to claim 1 for stabilizing organic material against oxidative, thermal or actinic degradation.

11. A process for stabilizing organic material against thermal, oxidative and/or actinic degradation, which comprises adding at least one compound of the formula I according to claim 1 to this material.

12. A compound obtainable by polycondensation or polyaddition of a compound of the formula II 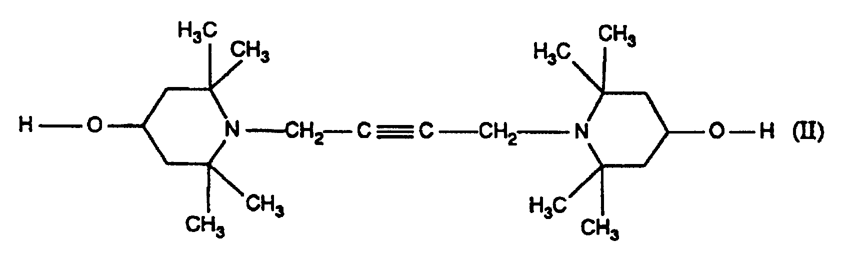 with a bifunctional reaction partner selected from dicarboxylic acids, dichlorides and diesters of dicarboxylic acids of the formula G-CO-R²-CO-G or G-CO-CO-G, in which G is hydrogen, Cl or C₁-C₄alkoxy; or an anhydride of the formula 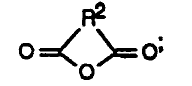 diisocyanates of the formula OCN-R⁵-NCO; dihalogen compounds of the formula Hal-R⁷-Hal, in which Hal is in each case a halogen atom; phosgene; C₁-C₄alkyl carbonic acid diesters; or bischloroformates of the Cl-CO-O-R⁶-O-CO-Cl type, in which
R², R⁵ and R⁷ are C₁-C₁₈alkylene; C₂-C₁₈alkylene which is interrupted by O or S; phenylene; a mixed aromatic/aliphatic radical containing 7 to 12 carbon atoms; naphthylene; or an -R⁴-D-R⁴- radical;
R⁴ is 1,4-cyclohexylene or 1,4-phenylene; R⁶ is phenylene, naphthylene or 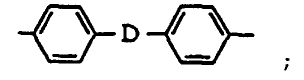 and
D is C₁-C₃alkylene, -Sr SO, -SO₂-, -CO-, -O-, -NH- or -NR¹-.

## Revendications

1. Composé de formule I 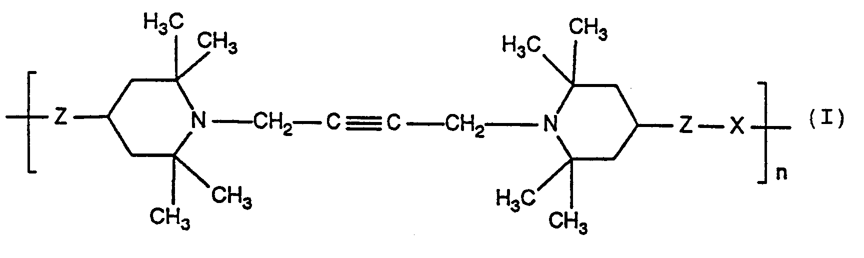 où
n est un nombre dans le domaine de 1 à 100 ;
X possède les significations de -R⁷-, -CO-, -CO-CO-, -CO-R²-CO-, -CO-NH-R⁵-NR-CO- et -CO-O-R⁶-O-CO- et 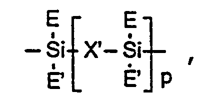 p étant un nombre dans le domaine de 0 à 6, X' représente un atome de O ou une liaison directe, E représente des groupes alkyle en C₁-C₁₂, phényle, phénylalkyle en C₇-C₉ ou cycloalkyle en C₅-C₁₂, et E' représente des atomes d'hydrogène ou possède l'une des significations données pour E ;
Z dans le cas où X est R⁷, représente O ou NR¹ ; Z, dans le cas où X est 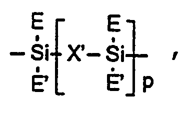 représente O ;
et
Z dans tous les autres cas représente O, NH ou NR¹ ;
R¹ représente des groupes alkyle en C₁-C₁₈, phénylalkyle en C₇-C₉, cycloalkyle en C₅-C₇, alcanoyle en C₂-C₈, benzoyle ou benzoyle substitué par alkyle en C₁-C₄ ;
R² et R⁷ représente des groupes alkylène en C₁-C₁₈ ; alkylène en C₂-C₁₈ interrompu par des atomes O ou S ; phénylène ; un reste mixte aromatic/aliphatique présentant 7 à 12 atomes de carbone ; naphtylène ; ou un reste -R⁴-D-R⁴- ;
R⁴ représente les groupes 1,4-cyclohexylène ou 1,4-phénylène ; et
R⁶ représente les groupes phénylène, naphtylène ou 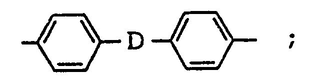
R⁵ dans le cas où Z représente O, possède l'une des significations données pour R² ; et
R⁵ dans le cas où Z représente NH ou NR¹, possède l'une des significations données pour R⁶ ; et
D représente les groupes alkylène en C₁-C₃, -S-, -SO-, -SO₂-, -CO-, -O-, -NH- ou NR¹-.

2. Composé de formule I, selon la revendication 1, où n représente un nombre dans le domaine de 4 à 80.

3. Composé de formule I, selon la revendication 1, où
X comprend les significations -CO-, -CO-CO-, -CO-R²-CO- 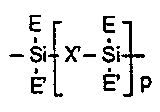 ou -CO-NH-R⁵-NH-CO-, et -CO-NH-R⁵-NH-CO-, p valant 0 ou 1, X' représente 0 ou une liaison directe, E' représente des atomes d'hydrogène, des groupes phényle ou méthyle et E représente phényle ou méthyle ;
R¹ représente des groupes alkyle en C₁-C₈, benzyle, alcanoyle en C₂-C₈, benzoyle ou cyclohexyle ;
R² et R⁷ représentent des groupes alkylène en C₁-C₁₈ ; aIkylène en C₂-C₁₈ interrompu par des atomes O ou S ; phénylène ; un reste mixte aromatique/aliphatique présentant 7 à 12 atomes de carbone : naphtylène ; ou un reste -R⁴-D-R⁴- ;
R⁴ représente les groupes 1,4-cyclohexylène ou 1,4-phénylène ; et
R⁵ dans le cas où Z représente O, représente les groupes alkylène en C₃-C₁₂, phénylène, naphtylène, cyclohexylène, tolylène ou éthylène interrompu par phénylène, ou -R⁴-CH₂-R⁴- ; et
D représente des groupes alkylène en C₁-C₃, -NH-, -NR¹- -CO- ou -S-.

4. Composé de formule I, selon la revendication 1, où
Z représente O ou NR¹ ;
X possède les significations -CO-CO-, -CO-R²-CO-, 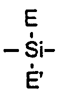 et -CO-NH-R⁵-NR-CO- ;
R² et R⁷ représentent alkylène en C₁-C₁₈ ; phénylène, CH₂-S-CH₂-, CH₂-O-CH₂-, ou 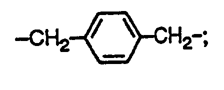 et
R⁵ dans le cas où Z est O, représente des groupes alkylène en C₃-C₁₂, phénylène, naphtylène, cyclohexylène, tolylène, éthylène interrompu par phénylène, ou -R⁴-CH₂-R⁴-, R⁴ représente les groupes 1,4-cyclohexylène ou 1,4-phénylène ; et R⁵, dans le cas où Z est NR¹, représente phénylène.

5. Composés de formule I selon la revendication 4, où
n est un nombre entier dans le domaine de 5 à 50 ;
Z représente un atome d'oxygène ;
R² et R⁷ représentent des groupes alkylène en C₁-C₁₂, phénylène, naphtylène ou cyclohexylène.

6. Composition contenant
A) une matière organique sensibles à la dégradation induite par l'oxygène, la chaleur ou/et un rayonnement actinique et
B) au moins un composé de formule I selon la revendication 1.

7. Composition selon la revendication 6 contenant comme composant A un polymère organique synthétique.

8. Composition selon la revendication 6 contenant de 0,01 à 10% du composé du composant B, par rapport à la matière à stabiliser.

9. Composition selon la revendication 6 contenant comme composant C supplémentaire un ou plusieurs additifs classiques.

10. Utilisation de composés de formule I selon la revendication 1 pour la stabilisation de matière organique contre la dégradation induite par l'oxygène, la chaleur ou un rayonnement actinique.

11. Procédé de stabilisation de matière organique contre la dégradation induite par l'oxygène, la chaleur ou un rayonnement actinique, caractérisé en ce que l'on ajoute à cette matière au moins un composé de formule I selon la revendication 1.

12. Composé que l'on peut obtenir par polycondensation ou par polyaddition d'un composé de formule II 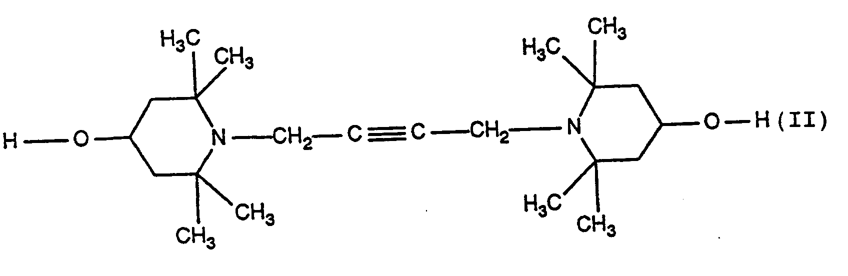 avec un partenaire de réaction bifonctionnel pris parmi les acides carboxyliques, les dichlorures ou les diesters d'acides carboxyliques de formule G-CO-R²-CO-G ou G-CO-CO-G où G représente un atome d'hydrogène, Cl ou alkoxy en C₁-C₄ ; ou un anhydride de formule 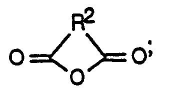 des diisocyantes de formule OCN-R⁵-NCO ; des composés dihalogénés de formule Hal-R⁷-Hal, où Hal représente chacun un atome d'halogène ; phosgène ; des esters d'alkyle en C₁-C₄ de l'acide carbonique ; ou des esters de l'acide bischloroformique du type Cl-CO-O-R⁶-O-CO-Cl, où
R², R⁵ et R⁷ représentent des groupes alkylène en C₁-C₁₈ ; alkylène en C₂-C₁₈ interrompu par des atomes de O ou S ; phénylène ; un reste aromatique/aliphatique mixte contenant de 7 à 12 atomes de carbone ; naphtylène ; ou un reste -R⁴-D-R⁴- ;
R⁴ représente les groupes 1,4-cyclohexylène ou 1,4-phénylène ;
R⁶ représente les groupes phénylène, naphtylène ou 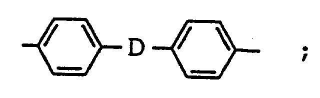 et
D représente des groupes alkylène en C₁-C₃, -S-, -SO-, -SO2-, -CO-, -O-, -NH- ou NR¹.
